# EUROPEAN PATENT APPLICATION

(11) **EP 2 085 120 A1**
(43) Date of publication of application: **05.08.2009**
(21) Application number: 08250397.0
(22) Date of filing: 01.02.2008
(51) Int. Cl.: A61P 25/28

(54) **The use of substances for the treatment of central or peripheral insulin receptor impairment and insulin resistance**

(71) Applicant: Merz Pharma GmbH & Co. KGaA, 60318 Frankfurt am Main (DE)
(72) Inventor: The designation of the inventor has not yet been filed
(74) Representative: Goddard, Christopher Robert

(57) **Abstract**

Methods for the prevention and treatment of conditions characterized by central or peripheral insulin deficiency, in particular such as resulting from insulin receptor impairment and insulin resistance, through blocking the toxic effects of β-amyloid (Aβ) derivatives, and pharmaceutical compositions for effecting such prevention and treatment thereof.

## Description

### FIELD OF THE INVENTION

The present invention is concerned with methods for the prevention and treatment of conditions characterized by central or peripheral insulin deficiency, in particular such as resulting from insulin receptor impairment and insulin resistance, through blocking the toxic effects of β-amyloid (Aβ) derivatives, and pharmaceutical compositions for effecting such prevention and treatment thereof.

### BACKGROUND OF THE INVENTION

Insulin receptors (IR) are receptor protein kinases, specifically tyrosine kinases. As tyrosine kinase receptors, IR have been associated with a broad spectrum of activities including cellular metabolism as well as signaling. The IR is characterized by an extracellular domain which is responsible for binding a ligand, such as insulin, a transmembrane domain, and an intracellular domain. IR are present both peripherally and in the CNS. Moreover, the function of IRs has been demonstrated to differ depending on location. Disruption in IR sensitivity has been linked to such diverse conditions as diabetes as well as Alzheimer's disease. For example, type 2 diabetes mellitus may be characterized, in part, by insulin insensitivity in which IR have diminished function in glucose metabolism. Recently, research has demonstrated that insulin insensitivity may be associated with the physiological circumstances resulting in or incident to Alzheimer's disease.

The toxic effects of β-amyloid (Aβ) derivatives have been associated with the Alzheimer's disease etiology. Aβ derivatives have been isolated in brain and cerebrospinal fluid of Alzheimer's disease patients in postmortem analysis, exhibiting levels uncharacteristic of patients not suffering from Alzheimer's disease. More specifically, Aβ derived diffusible ligands (ADDLs) have been isolated in postmortem analysis of patients suffering from Alzheimer's disease. Based on these findings, it has now been demonstrated that ADDLs induce significant changes in synaptic plasticity in isolated hippocampal and cortical cultured neurons. LACOR, P.-N., BUNIEL, M.C., FURLOW, P.W., CLEMENTE, A.S., VELASCO, P.T., WOOD, M., VIOLA, K.L. and KLEIN, W.L. (2007) Aβ oligomers-induced aberrations in synapse composition, shape and density provide a molecular basis for loss of connectivity in Alzheimer's disease. J. Neurosci. 27: 796-807. ZHAO go on to demonstrate that insulin sensitivity is significantly diminished upon pretreating cultured hippocampal and cortical neurons with ADDLs. Wei-Qin ZHAO, Fernanda G. DE FELICE, Sara FERNANDEZ, Hui CHEN, Mary P. LAMBERT, Michael J. QUON, Grant A. KRAFFT, and William L. KLEIN, Amyloid beta oligomers induce impairment of neuronal insulin receptors, The FASEB Journal 22 (February 2008) Moreover, combined ADDL/glutamate stimulation of cultured cells also resulted in decreased sensitivity. Both the ADDL and glutamate-induced decreased insulin sensitivity were demonstrated to be reversible by the addition of an NMDA receptor antagonist, for example memantine, resulting in the conclusion that IR sensitivity, or rather ADDL modulation is also connected to NMDA and other synaptic receptors.

### OBJECTS OF THE INVENTION

It is an object of the present invention to provide novel methods for the prevention and treatment of conditions characterized by central or peripheral insulin deficiency, in particular such as resulting from insulin receptor impairment and insulin resistance, and pharmaceutical compositions for effecting such prevention and treatment thereof. Yet additional objects will become apparent hereinafter, and still further objects will be apparent to one skilled in the art.

### SUMMARY OF THE INVENTION

What we therefore believe to be comprised by our invention may be summarized *inter alia* in the following words:

A method-of-treating a living animal, including a human, for the prevention and treatment of conditions characterized by central or peripheral neuronal insulin receptor impairment and insulin resistance, which conditions may be associated with β-amyloid (Aβ) toxicity, comprising the step of administering to the living animal a therapeutically effective amount of a peptide comprising amino acid sequence X-Y or Y-X, wherein X is an aromatic amino acid and Y is one or more additional amino acid other than glycine, the peptide having at least 2 amino acid residues and less than 15 amino acid residues, thereby inhibiting Aβ formation and/or occurrence of said Aβ, which is effective for alleviation of the condition.

Such a method wherein the condition characterized by central or peripheral neuronal insulin receptor impairment and insulin resistance is selected from the group consisting of amyotrophic lateral sclerosis, Alzheimer's disease, Parkinson's disease, Huntington's disease, schizophrenia, fronto-temporal dementia, Nieman-Pick disease Type C, obesity, depressive disorders, spinocerebellar ataxia, progressive supramuscular palsy, multiple sclerosis, Guillain-Barré syndrome, Charcot-Marie-Tooth syndrome, insulin receptor impairment, Cushing's disease, Donohue syndrome, polycystic ovarian syndrome, haemochromatosis, hypertension, HIV infection and tumors.

Such a method comprising the step of co-administering to the living animal a therapeutically effective amount of a peptide as described above in combination with at least one additional pharmaceutical agent which is effective in treating progressive neurodegenerative and metabolic disorders, wherein the combination of the peptide and the at least one additional pharmaceutical agent is effective in treating the condition.

Such a method wherein the at least one additional pharmaceutical agent is selected from medications administered to prevent or treat progressive neurodegenerative and metabolic disorders, such additional pharmacteutical agent being selected from other β-amyloid aggregation inhibitors (GAG inhibitors, β-sheet inhibitors, QC inhibitors), β-amyloid inhibitors (e.g. γ-secretase inhibitors, β-secretase inhibitors, α-secretase activators, glutaminyl-cyclase-inhibitors, APP expression inhibitors, passive and active vaccination / immunization), phosphorylated tau modulators (e.g. GSK-3 and CDK-5 inhibitors), PPARγ modulators, glutamate modulators (e.g. NMDA receptor antagonists, NR2B antagonists, glycine_{B} antagonists /agonists, AMPAkines, metabotropic glutamate receptor group I agonists /positive allosteric modulators, metabotropic glutamate receptor group II/III antagonists / negative allosteric modulators, glutamate release modulators), cannabinoid modulators (e.g. CB1 receptor antagonists, CB2 receptor agonists), GABA_{B} receptor antagonists, acetylcholine-esterase inhibitors, acetylcholine receptor modulators (α7 and α4β2 nicotinic receptor agonists, M1 receptors agonists, M2 receptor antagonists), serotonin modulators (e.g. 5-HT_{1A} and 5-HT₆ receptor antagonists, 5-HT₄ receptor agonists), GNRH agonists, histamine modulators (e.g. H1 and H3 receptor antagonists), apoptosis / necrosis inhibitors, Cox inhibitors, dopamine modulators (e.g. D1 receptor agonists), adenosine modulators (e.g. A1 / A2A receptor antagonists), phosphodiesterase modulators (e.g. PDE4 inhibitors), Cu²⁺/Zn²⁺ chelators, Ca²⁺ channel antagonists, statins, NSAIDs.

Such a method wherein the at least one additional pharmaceutical agent is selected from ABT-089, ACC-001, Alzhemed, Ampalex, Ave-1625, AZD-103, Babineuzumab, CAD-106, Celebrex, D-cycloserine, Dimebon, Donepezil, D-serine, Flurizan, Galanthamine, Glycine, GSK-189254, GSK742457, Isopronicline, Lecozotan, Leuprolide, m266, MEM-3454, Memantine, Neramexane, NP-031122, Ono-2506, Pioglitazone, Posiphen, PRX-03140, Riluzole, Rivastigmine, Rosiglitazone, SGS-742.

Such a method wherein the peptide is administered once a day, twice a day or three times a day.

Such a method wherein the peptide is administered chronically.

Such a method wherein the peptide is administered orally.

Such a,method wherein the peptide is administered in an immediate or modified release formulation.

Such a method wherein the peptide and the at least one additional pharmaceutical agent are administered conjointly.

Such a method wherein the peptide and the at least one additional pharmaceutical agent are administered in a single formulation.

A further aspect of the invention relates to a pharmaceutical composition comprising a peptide as described above, or a pharmaceutically acceptable addition salt thereof, alone or in combination with one or more pharmaceutically acceptable carrier and/or excipient.

Such a pharmaceutical composition further comprising at least one additional pharmaceutical agent which is effective in treating progressive neurodegenerative and metabolic disorders, wherein the combination of the peptide and the at least one additional pharmaceutical agent is effective in treating the condition, or a pharmaceutically acceptable addition salt thereof, alone or in combination with one or more pharmaceutically acceptable carrier and/or excipient.

A further aspect of the invention relates to the use of peptide comprising amino acid sequence X-Y or Y-X, wherein X is an aromatic amino acid and Y is one or more additional amino acid other than glycine, the peptide having at least 2 amino acid residues and less than 15 amino acid residues, for the manufacture of a medicament for the prevention and treatment of a condition characterized by central or peripheral neuronal insulin receptor impairment and insulin resistance associated with β-amyloid (Aβ) toxicity.

Such a use characterized in that the condition characterized by central or peripheral neuronal insulin receptor impairment and insulin resistance is selected from the group consisting of amyotrophic lateral sclerosis, Alzheimer's disease, Parkinson's disease, Huntington's disease, schizophrenia, fronto-temporal dementia, Nieman-Pick disease Type C, obesity, depressive disorders, spinocerebellar ataxia, progressive supramuscular palsy, multiple sclerosis, Guillain-Barré syndrome, Charcot-Marie-Tooth syndrome, insulin receptor impairment, Cushing's disease, Donohue syndrome, polycystic ovarian syndrome, haemochromatosis, hypertension, HIV infection and tumors.

Such a use characterized in that the medicament comprises the above-identified peptide in combination with at least one additional pharmaceutical agent which is effective in treating progressive neurodegenerative and metabolic disorders, further characterized in that the additional pharmaceutical agent is selected from other β-amyloid aggregation inhibitors (GAG inhibitors, β-sheet inhibitors, QC inhibitors), β-amyloid inhibitors (e.g. γ-secretase inhibitors, β-secretase inhibitors, α-secretase activators, glutaminyl-cyclase-inhibitors, APP expression inhibitors, passive and active vaccination /immunization), phosphorylated tau modulators (e.g. GSK-3 and CDK-5 inhibitors), PPARγ modulators, glutamate modulators (e.g. NMDA receptor antagonists, NR2B antagonists, glycine_{B} antagonists / agonists, AMPAkines, metabotropic glutamate receptor group I agonists / positive allosteric modulators, metabotropic glutamate receptor group II/III antagonists /negative allosteric modulators, glutamate release modulators), cannabinoid modulators (e.g. CB1 receptor antagonists, CB2 receptor agonists), GABA_{B} receptor antagonists, acetylcholine-esterase inhibitors, acetylcholine receptor modulators (α7 and α4β2 nicotinic receptor agonists, M1 receptors agonists, M2 receptor antagonists), serotonin modulators (e.g. 5-HT_{1A} and 5-HT₆ receptor antagonists, 5-HT₄ receptor agonists), GNRH agonists, histamine modulators (e.g. H1 and H3 receptor antagonists), apoptosis / necrosis inhibitors, Cox inhibitors, dopamine modulators (e.g. D1 receptor agonists), adenosine modulators (e.g. A1 / A2A receptor antagonists), phosphodiesterase modulators (e.g. PDE4 inhibitors), Cu²⁺/Zn²⁺ chelators, Ca²⁺ channel antagonists, statins, NSAIDs.

### DETAILED DESCRIPTION OF THE INVENTION

The present invention is concerned with methods for the prevention and treatment of conditions characterized by central or peripheral insulin deficiency, in particular such as resulting from insulin receptor impairment and insulin resistance, through blocking the toxic effects of β-amyloid (Aβ) derivatives through the administration of novel peptides as described herein, alone or in conjunction with other substances recognized to be useful in treating such conditions.

Representative substances described for the instant therapy were initially designed for the treatment of diseases characterized by formation of amyloid fibrils, such as type II diabetes and prion diseases [Porat Y, Mazor Y, Efrat S, Gazit E. Inhibition of islet amyloid polypeptide fibril formation : A potential role for heteroaromatic interactions. Biochemistry 2004; 43:14454-14462], and degenerative diseases of the brain including Alzheimer's dementia [GAZIT, E., US Published Application No. US2006/0234947 A1]. More specifically, Gazit discloses that short chain peptides, possibly comprising modified amino acids such as aminoisobutyric acid, have application in the disruption of the formation of toxic Aβ species by interacting with molecular recognition processes and amyloid fibril self assembly. [Gazit, 2006] With the instant invention, we have determined that these substances show therapeutic effects in the prevention and treatment of condition characterized by central or peripheral neuronal insulin receptor impairment and insulin resistance.

Methods comprising the administration of these substances find application in treating patients with all types of conditions characterized by central or peripheral neuronal insulin receptor impairment and insulin resistance, as aforementioned, and amyotrophic lateral sclerosis, Alzheimer's disease, Parkinson's disease, Huntington's disease, schizophrenia, fronto-temporal dementia, Nieman-Pick disease Type C, obesity, depressive disorders, spinocerebellar ataxia, progressive supramuscular palsy, multiple sclerosis, Guillain-Barré syndrome, Charcot-Marie-Tooth syndrome, insulin receptor impairment, Cushing's disease, Donohue syndrome, polycystic ovarian syndrome, haemochromatosis, hypertension, HIV infection and tumors. Treatment is possible at all stages of disease progression including very early stages as a prophylactic. The clinical effects may be two-fold, first an acute improvement neural function in those patients already suffering from a neurodegeneration, and second a slowing-down or stopping of the progressive neurodegeneration.

The instant methods optionally comprise administering preparations containing these substances on a continuous basis to provide best treatment outcomes. Depending on the type and the stage of the underlying disease treatment, cycles of some days to several months are possible. Under certain conditions also continuous long-term treatment is possible.

The instant methods optionally comprise administering at least one additional pharmaceutical agent which is known in the art to be effective in the treatment of progressive neurodegenerative and metabolic disorders. These additional agents may be selected from the general class of other β-amyloid aggregation inhibitors (GAG inhibitors, β-sheet inhibitors, QC inhibitors), β-amyloid inhibitors (e.g. γ-secretase inhibitors, β-secretase inhibitors, α-secretase activators, glutaminyl-cyclase-inhibitors, APP expression inhibitors, passive and active vaccination / immunization), phosphorylated tau modulators (e.g. GSK-3 and CDK-5 inhibitors), PPARγ modulators, glutamate modulators (e.g. NMDA receptor antagonists, NR2B antagonists, glycine_{B} antagonists / agonists, AMPAkines, metabotropic glutamate receptor group I agonists / positive allosteric modulators, metabotropic glutamate receptor group II/III antagonists / negative allosteric modulators, glutamate release modulators), cannabinoid modulators (e.g. CB1 receptor antagonists, CB2 receptor agonists), GABA_{B} receptor antagonists, acetylcholine-esterase inhibitors, acetylcholine receptor modulators (α7 and α4β2 nicotinic receptor agonists, M1 receptors agonists, M2 receptor antagonists), serotonin modulators (e.g. 5-HT_{1A} and 5-HT₆ receptor antagonists, 5-HT₄ receptor agonists), GNRH agonists, histamine modulators (e.g. H1 and H3 receptor antagonists), apoptosis / necrosis inhibitors, Cox inhibitors, dopamine modulators (e.g. D1 receptor agonists), adenosine modulators (e.g. A1 / A2A receptor antagonists), phosphodiesterase modulators (e.g. PDE4 inhibitors), Cu²⁺/Zn²⁺ chelators, Ca²⁺ channel antagonists, statins, NSAIDs. Representative substances include ABT-089, ACC-001, Alzhemed, Ampalex, Ave-1625, AZD-103, Babineuzumab, CAD-106, Celebrex, D-cycloserine, Dimebon, Donepezil, D-serine, Flurizan, Galanthamine, Glycine, GSK-189254, GSK742457, Isopronicline, Lecozotan, Leuprolide, m266, MEM-3454, Memantine, Neramexane, NP-031122, Ono-2506, Pioglitazone, Posiphen, PRX-03140, Riluzole, Rivastigmine, Rosiglitazone, SGS-742

The following peptides as described in US Published Application No. US2006/0234947 A1 are representative of those substances which are active in the methods of the present invention. D-Phe-D-Phe-D-Pro (SEQ ID NO. 1), Aib-D-Phe-D-Asn-Aib (SEQ ID NO. 2), D-Phe-D-Asn-D-Pro (SEQ ID NO. 3), Aib-Asn-Phe-Aib (SEQ ID NO. 4), Gln-Lys-Leu-Val-Phe-Phe (SEQ ID NO. 5), Tyr-Tyr (SEQ ID NO. 6), D-Phe-D-Phe-D-Pro (SEQ ID NO. 7), Aib-D-Phe-D-Asn-Aib (SEQ ID NO. 8), Aib-Asn-Phe-Aib (SEQ ID NO. 9), Tyr-Tyr (SEQ ID NO. 10), Tyr-Tyr-NH₂ (SEQ ID NO. 11), Aib-Phe-Phe (SEQ ID NO. 12), Asn-Tyr-Aib (SEQ ID NO. 13), Asn-Tyr-Pro (SEQ ID NO. 14), β-aminoisobutyric acid (Aib)-D-Pro-D-Tyr-D-Asn (SEQ ID NO. 15), D-Tyr-Aib (SEQ ID NO. 16), D-Pro-D-Tyr (SEQ ID NO. 17), D-Tyr-D-Pro (SEQ ID NO. 18), Asn-Tyr-Tyr-Pro (SEQ ID NO. 19), Tyr-Tyr-Aib (SEQ ID NO. 20), Aib-Tyr-Tyr (SEQ ID NO. 21), Aib-Tyr-Tyr-Aib (SEQ ID NO. 22), D-Asn-Tyr-Tyr-D-Pro (SEQ ID NO. 23), Pro-Tyr-Tyr (SEQ ID NO. 24), Tyr-Tyr-Pro (SEQ ID NO. 25), Pro-Tyr-Tyr-Pro (SEQ ID NO. 26), D-Tyr-D-Tyr (SEQ ID NO. 27), D-Pro-Aib (SEQ ID NO. 28), D-Phe-D-Pro (SEQ ID NO. 29), D-Trp-Aib (SEQ ID NO. 30), D-Trp-D-Pro (SEQ ID NO. 31), D-Phe-Pro (SEQ ID NO. 32), and Pro-D-Phe (SEQ ID NO. 33). Unless otherwise noted, the residue Aib is understood to mean α aminoisobutyric acid.

It will be apparent to those skilled in the art that the described substances are merely representative in nature and that alternative substances are known to one of ordinary skill in pharmacology.

The term "analog" or "derivative" is used herein in the conventional pharmaceutical sense, to refer to a molecule that structurally resembles an active substance of the instant method (such as D-Trp-Aib), but has been modified in a targeted and controlled manner to replace one or more specific substituents of the referent molecule with an alternate substituent, thereby generating a molecule which is structurally similar to the reference molecule. Synthesis and screening of analogs (e.g., using structural and/or biochemical analysis), to identify slightly modified versions of a known substance which may have improved or biased traits (such as higher potency and/or selectivity at a specific targeted receptor type, greater ability to penetrate mammalian blood-brain barriers, fewer side effects, etc.) is a drug design approach that is well known in pharmaceutical chemistry.

In addition, using methods known to those skilled in the art, analogs and derivatives of the substances of the invention can be created which have improved therapeutic efficacy in controlling β-amyloid (Aβ) toxicity, *i*.*e*., higher potency and/or selectivity at a specific targeted receptor type, either greater or lower ability to penetrate mammalian blood-brain barriers (e.g., either higher or lower blood-brain barrier permeation rate), fewer side effects, etc.

Due to their high degree of activity and their low toxicity, together presenting a most favorable therapeutic index, the substances of the invention may be administered to a subject, e.g., a living animal (including a human) body, in need thereof, for the treatment, alleviation, or amelioration, palliation, or elimination of an indication or condition which is susceptible thereto, or representatively of an indication or condition set forth elsewhere in this application, preferably concurrently, simultaneously, or together with one or more pharmaceutically-acceptable excipients, carriers, or diluents, especially and preferably in the form of a pharmaceutical composition thereof, whether by oral, rectal, or parental (including intravenous, intraocular, and subcutaneous) or in some cases even topical route (including eye drops, eye creams, and intraocular depot formulations), in an effective amount. Suitable dosage ranges include 1-1000 milligrams daily, alternately 10-500 milligrams daily, and optionally 50-500 milligrams daily, depending as usual upon the exact mode of administration, form in which administered, the indication toward which the administration is directed, the subject involved and the body weight of the subject involved, and the preference and experience of the physician or veterinarian in charge.

The term "therapeutically effective" applied to dose or amount refers to that quantity of a substance or pharmaceutical composition that is sufficient to result in a desired activity upon administration to a living animal body (including a human body) in need thereof. The term "treat" is used herein to mean to relieve or alleviate at least one symptom of a disease in a subject. Within the meaning of the present invention, the term "treat" also denotes to arrest, delay the onset (*i*.*e*., the period prior to clinical manifestation of a disease) and/or reduce the risk of developing or worsening a disease.

The phrase "pharmaceutically acceptable", as used in connection with compositions of the invention, refers to molecular entities and other ingredients of such compositions that are physiologically tolerable and do not typically produce untoward reactions when administered to a mammal (e.g., human). The term "pharmaceutically acceptable" may also mean approved by a regulatory agency of the Federal or a state government or listed in the U.S. Pharmacopeia or other generally recognized pharmacopeia for use in mammals, and more particularly in humans.

Substances of the present invention may be in the form of pharmaceutically acceptable salts. "Pharmaceutically acceptable salts" refers to those salts which possess the biological effectiveness and properties of the parent compound and which are not biologically or otherwise undesirable. The nature of the salt or isomer is not critical, provided that it is non-toxic and does not substantially interfere with the desired pharmacological activity.

The term "carrier" applied to pharmaceutical compositions of the invention refers to a diluent, excipient, or vehicle with which an active substance (such as D-Trp-Aib) is administered. Such pharmaceutical carriers may be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by A.R. Gennaro, 20^{th} Edition.

The term "about" or "approximately" usually means within 20%, alternatively within 10%, including within 5% of a given value or range. Alternatively, especially in biological systems, the term "about" means within about a log (i.e., an order of magnitude), including within a factor of two of a given value.

In conjunction with the methods of the present invention, also provided are pharmaceutical compositions comprising a therapeutically effective amount of the active substance. The compositions of the invention may further comprise a carrier or excipient (all pharmaceutically acceptable). The compositions may be formulated for once-a-day administration, twice-a-day administration, or three times a day administration.

According to the present invention, the dosage form of the active substance may be a solid, semisolid, or liquid formulation according to the following.

The active substance of the instant invention may be administered orally, topically, parenterally, or mucosally (e.g., buccally, by inhalation, or rectally) in dosage unit formulations containing conventional non-toxic pharmaceutically acceptable carriers. In another embodiment for administration to pediatric subjects, the active substance may be formulated as a flavored liquid (e.g., peppermint flavor). The active substance may be administered orally in the form of a capsule, a tablet, or the like, or as a semi-solid, or liquid formulation (see Remington's Pharmaceutical Sciences, 20^{th} Edition, by A.R. Gennaro).

For oral administration in the form of a tablet or capsule, the active substance may be combined with non-toxic, pharmaceutically acceptable excipients such as binding agents (e.g., pre-gelatinized maize starch, polyvinylpyrrolidone or hydroxypropyl methylcellulose); fillers (e.g., lactose, sucrose, glucose, mannitol, sorbitol and other reducing and non-reducing sugars, microcrystalline cellulose, calcium sulfate, or calcium hydrogen phosphate); lubricants (e.g., magnesium stearate, talc, or silica, steric acid, sodium stearyl fumarate, glyceryl behenate, calcium stearate, and the like); disintegrants (e.g., potato starch or sodium starch glycolate); or wetting agents (e.g., sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like.

The tablets may be coated with a concentrated sugar solution which may contain e.g., gum arabic, gelatine, talcum, titanium dioxide, and the like. Alternatively, the tablets may be coated with a polymer that dissolves in a readily volatile organic solvent or mixture of organic solvents. In specific embodiments, the active substance is formulated into immediate-release or modified-release tablets. Immediate release solid dosage forms permit the release of most or all of the active ingredient over a short period of time, such as 60 minutes or less, and make rapid absorption of the drug possible. Modified release solid oral dosage forms permit the sustained release of the active substance over an extended period of time in an effort to maintain therapeutically effective plasma levels over similarly extended time intervals and/or to modify other pharmacokinetic properties of the active substance.

For the formulation of soft gelatin capsules, the active substances may be admixed with e.g., a vegetable oil or poly-ethylene glycol. Hard gelatin capsules may contain granules of the active substances using either the above mentioned excipients for tablets e.g., lactose, saccharose, sorbitol, mannitol, starches (e.g., potato starch, corn starch or amylopectin), cellulose derivatives or gelatine. Also liquids or semi-solids of the drug may be filled into hard gelatine capsules.

The compositions of the invention may also be introduced in microspheres or microcapsules, e.g., fabricated from polyglycolic acid/lactic acid (PGLA) (see, e.g., U.S. Patents No. 5,814,344; 5,100,669 and 4,849,222; PCT Publications No. WO 95/11010 and WO 93/07861). Biocompatible polymers may be used in achieving controlled release of an active substance, include for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and crosslinked or amphipathic block copolymers of hydrogels.

Formulation of an active substance in a semi-solid or liquid form may also be used. The substance may constitute between 0.1 and 99% by weight of the formulation, more specifically between 0.5 and 20% by weight for formulations intended for injection and between 0.2 and 50% by weight for formulations suitable for oral administration.

In one embodiment of the invention, the active substance is administered in a modified release formulation. Modified release dosage forms provide a means for improving patient compliance and for ensuring effective and safe therapy by reducing the incidence of adverse drug reactions. Compared to immediate release dosage forms, modified release dosage forms can be used to prolong pharmacologic action after administration, and to reduce variability in the plasma concentration of a drug throughout the dosage interval, thereby eliminating or reducing sharp peaks.

A modified release dosage form may comprise a core either coated with or containing an active substance. The core being is then coated with a release modifying polymer within which the active substance is dispersed. The release modifying polymer disintegrates gradually, releasing the active substance over time. Thus, the outer-most layer of the composition effectively slows down and thereby regulates the diffusion of the active substance across the coating layer when the composition is exposed to an aqueous environment, i.e. the gastrointestinal tract. The net rate of diffusion of the active substance is mainly dependent on the ability of the gastric fluid to penetrate the coating layer or matrix and on the solubility of the active substance itself.

In another embodiment of the invention, the active substance is formulated in an oral, liquid formulation. Liquid preparations for oral administration can take the form of, for example, solutions, syrups, emulsions or suspensions, or they can be presented as a dry product for reconstitution with water or other suitable vehicle before use. Preparations for oral administration can be suitably formulated to give controlled or postponed release of the active compound.

For oral administration in liquid form, the active substance may be combined with non-toxic, pharmaceutically acceptable inert carriers (e.g., ethanol, glycerol, water), suspending agents (e.g., sorbitol syrup, cellulose derivatives or hydrogenated edible fats), emulsifying agents (e.g., lecithin or acacia), non-aqueous vehicles (e.g., almond oil, oily esters, ethyl alcohol or fractionated vegetable oils), preservatives (e.g., methyl or propyl-p-hydroxybenzoates or sorbic acid), and the like. Stabilizing agents such as antioxidants (BHA, BHT, propyl gallate, sodium ascorbate, citric acid) may also be added to stabilize the dosage forms. For example, solutions may contain from about 0.2% to about 20% by weight of the active substance, with the balance being sugar and mixture of ethanol, water, glycerol and propylene glycol. Optionally, such liquid formulations may contain coloring agents, flavoring agents, saccharine and carboxymethyl-cellulose as a thickening agent or other excipients.

In another embodiment, a therapeutically effective amount of the active substance is administered in an oral solution containing a preservative, a sweetener, a solubilizer, and a solvent. The oral solution may include one or more buffers, flavorings, or additional excipients. In a further embodiment, a peppermint or other flavoring is added to the active substance oral liquid formulation.

For administration by inhalation, the active substance may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide, or other suitable gas. In the case of a pressurized aerosol, the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

Solutions for parenteral applications by injection may be prepared in an aqueous solution of a water-soluble pharmaceutically acceptable salt of the active substances, preferably in a concentration of from about 0.5% to about 10% by weight. These solutions may also contain stabilizing agents and/or buffering agents and may conveniently be provided in various dosage unit ampoules.

The formulations of the invention may be delivered parenterally, i.e., by intraocular, intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection can be presented in unit dosage form, e.g., in ampoules or in multi-dose containers, with an added preservative. Alternatively, the active ingredient may be in powder form for reconstitution with a suitable vehicle, e.g., sterile pyrogen-free water, before use.

The invention also provides a pharmaceutical pack or kit comprising one or more containers containing the active substance and, optionally, more of the ingredients of the formulation. In a specific embodiment, the active substance is provided as an oral solution (for example 2 mg/ml) for administration with the use of a 2 teaspoon capacity syringe (dosage KORC®). Each oral syringe has hatch marks for measurement, with lines on the right side of the syringe (tip down) representing tsp units, and those on the left representing ml units.

The optimal therapeutically effective amount may be determined experimentally, taking into consideration the exact mode of administration, from in which the drug is administered, the indication toward which the administration is directed, the subject involved (e.g., body weight, health, age, sex, etc.), and the preference and experience of the physician or veterinarian in charge.

Dosage units for rectal application may be solutions or suspensions or may be prepared in the form of suppositories or retention enemas comprising the substances of the invention in a mixture with a neutral fatty base, or gelatin rectal capsules comprising the active substances in admixture with vegetable oil or paraffin oil.

Toxicity and therapeutic efficacy of the compositions of the invention may be determined by standard pharmaceutical procedures in experimental animals, e.g., by determining the LD50 (the dose lethal to 50% of the population) and the ED50 (the dose therapeutically effective in 50% of the population). The dose ratio between therapeutic and toxic effects is the therapeutic index and it may be expressed as the ratio LD50/ED50. Compositions that exhibit large therapeutic indices are preferred.

Suitable daily doses of the active substance of the invention in therapeutic treatment of humans are about 0.01-10 mg/kg bodyweight on peroral administration and 0.001-10 mg/kg bodyweight on parenteral administration.

Treatment duration may be short-term, e.g., several weeks (for example 8-14 weeks), or long-term until the attending physician deems further administration no longer is necessary.

The active substance of the instant invention may be administered as a monotherapy, or in combination with another substance prescribed for the treatment of progressive neurodegenerative and metabolic disorders.

The term "combination" applied to active substances is used herein to define a single pharmaceutical composition (formulation) comprising two active substances (e.g., a pharmaceutical composition comprising an active substance as described herein and another substance prescribed for the treatment of progressive neurodegenerative and metabolic disorders or two separate pharmaceutical compositions, each comprising an active substance (e.g. a pharmaceutical composition comprising a an active substance of the instant invention or another substance prescribed for the treatment of progressive neurodegenerative and metabolic disorders, to be administered conjointly.

Within the meaning of the present invention, the term "conjoint administration" is used to refer to administration of an active substance as described herein and a second active substance (e.g. another substance prescribed for the treatment of progressive neurodegenerative and metabolic disorders simultaneously in one composition, or simultaneously in different compositions, or sequentially. For the sequential administration to be considered "conjoint", however, an active substance as described herein and the second active substance must be administered separated by a time interval which still permits the resultant beneficial effect for treating progressive neurodegenerative and metabolic disorders in a mammal.

### PHARMACOLOGY - SUMMARY

The method of using the active substance of the present invention, and pharmaceutical compositions thereof, are characterized by unique advantageous and unpredictable properties, rendering the "subject matter as a whole", as claimed herein, unobvious. The methods and pharmaceutical compositions therefore have exhibited, in standard accepted reliable test procedures, the following valuable properties and characteristics:

### METHODS

Inhibition of neuronal insulin receptor Tyr phosphorylation (pTyr) by ADDLs. Hippocampal and cortical neuronal cultures (14-20 DIV) prepared from postnatal day 1 rats were "starved" in B27-free Neurobasal basal medium for 3-4 h and treated with 50 nM ADDLs / vehicle / active substance in the presence and absence of insulin (100 nM). IR was immunoprecipitated with an IRβ antibody and the pTyr detected on Western blots with antipTyr antibodies (4G10 and Py20).

### RESULTS

Thus, in these studies, the active substance of the present invention ameliorated the inhibition of neuronal insulin receptor Tyr phosphorylation (pTyr) by ADDLs. These data strongly suggest that the active substance of the present invention could be useful for the treatment of diseases asscoaueted with disturbances of neuronal insulin receptor function by β-amyloid peptides such as ADDLs.

### CONCLUSIONS

In conclusion, from the foregoing, it is apparent that the present invention provides novel, valuable, and surprising applications and uses for substances in the methods of the present invention, as well as novel pharmaceutical compositions thereof, all possessed of the foregoing more specifically-enumerated characteristics and advantages.

The high order of activity of the described methods for using the active substances of the present invention and compositions thereof, as evidenced by the tests reported, is indicative of utility. Clinical evaluation in human beings has not been completed, however. It will be clearly understood that the distribution and marketing of any substance or composition falling within the scope of the present invention for use in human beings will of course have to be predicated upon prior approval by governmental agencies, such as the U.S. Federal Food and Drug Administration, which are responsible for and authorized to pass judgment on such questions.

The method of treating a living animal body with a substance of the invention, for the inhibition of progression or alleviation of the selected ailment therein, is as previously stated by any normally-accepted pharmaceutical route, employing the selected dosage which is effective in the alleviation of the particular ailment desired to be alleviated.

Use of the substances of the present invention in the manufacture of a medicament for the treatment of a living animal for inhibition of progression or alleviation of selected ailments or conditions, particularly ailments or conditions such as treatment of conditions characterized by central or peripheral insulin deficiency, in particular such as resulting from insulin receptor impairment and insulin resistance, through blocking the toxic effects of β-amyloid (Aβ) derivatives, is carried out in the usual manner comprising the step of admixing an effective amount of a compound of the invention with a pharmaceutically-acceptable diluent, excipient, or carrier, and the method-of-treating, pharmaceutical compositions, and use of a compound of the present invention in the manufacture of a medicament.

Representative pharmaceutical compositions prepared by admixing the active substance with a suitable pharmaceutically-acceptable excipient, diluent, or carrier, include tablets, capsules, solutions for injection, liquid oral formulations, aerosol formulations, TDS formulations, and nanoparticle formulations, thus to produce medicaments for oral, injectable, or topical use, also in accord with the foregoing.

The present invention is not to be limited in scope by the specific embodiments described herein. Indeed, various modifications of the invention in addition to those described herein will become apparent to those skilled in the art from the foregoing description.

All patents, applications, publications, test methods, literature, and other materials cited herein are hereby incorporated by reference.

## Claims

1. Use of a peptide comprising amino acid sequence X-Y or Y-X, wherein X is an aromatic amino acid and Y is one or more additional amino acid other than glycine, the peptide having at least 2 amino acid residues and less than 15 amino acid residues, for the manufacture of a medicament for the prevention and treatment of a condition **characterized by** central or peripheral neuronal insulin receptor impairment and insulin resistance associated with β-amyloid (Aβ) toxicity.

2. The use of Claim 1, **characterized in that** the condition **characterized by** central or peripheral neuronal insulin receptor impairment and insulin resistance is selected from the group consisting of amyotrophic lateral sclerosis, Alzheimer's disease, Parkinson's disease, Huntington's disease, schizophrenia, fronto-temporal dementia, Nieman-Pick disease Type C, obesity, depressive disorders, spinocerebellar ataxia, progressive supramuscular palsy, multiple sclerosis, Guillain-Barré syndrome, Charcot-Marie-Tooth syndrome, insulin receptor impairment, Cushing's disease, Donohue syndrome, polycystic ovarian syndrome, haemochromatosis, hypertension, HIV infection and tumors.

3. The use of Claim 1, **characterized in that** the medicament comprises the peptide in combination with at least one additional pharmaceutical agent which is **characterized** as being effective in treating progressive neurodegenerative and metabolic disorders.

4. The use of Claim 3, wherein the at least one additional pharmaceutical agent is selected from medications **characterized** as being useful in preventing and treating progressive neurodegenerative and metabolic disorders, such medications being selected from other β-amyloid aggregation inhibitors (GAG inhibitors, β-sheet inhibitors, QC inhibitors), β-amyloid inhibitors (e.g. γ-secretase inhibitors, β-secretase inhibitors, α-secretase activators, glutaminyl-cyclase-inhibitors, APP expression inhibitors, passive and active vaccination / immunization), phosphorylated tau modulators (e.g. GSK-3 and CDK-5 inhibitors), PPARγ modulators, glutamate modulators (e.g. NMDA receptor antagonists, NR2B antagonists, glycine_{B} antagonists / agonists, AMPAkines, metabotropic glutamate receptor group I agonists / positive allosteric modulators, metabotropic glutamate receptor group II/III antagonists / negative allosteric modulators, glutamate release modulators), cannabinoid modulators (e.g. CB1 receptor antagonists, CB2 receptor agonists), GABA_{B} receptor antagonists, acetylcholine-esterase inhibitors, acetylcholine receptor modulators (α7 and α4β2 nicotinic receptor agonists, M1 receptors agonists, M2 receptor antagonists), serotonin modulators (e.g. 5-HT_{1A} and 5-HT₆ receptor antagonists, 5-HT₄ receptor agonists), GNRH agonists, histamine modulators (e.g. H1 and H3 receptor antagonists), apoptosis / necrosis inhibitors, Cox inhibitors, dopamine modulators (e.g. D1 receptor agonists), adenosine modulators (e.g. A1 / A2A receptor antagonists), phosphodiesterase modulators (e.g. PDE4 inhibitors), Cu²⁺/Zn²⁺ chelators, Ca²⁺ channel antagonists, statins, NSAIDs.

5. The use of Claim 3, wherein the at least one additional pharmaceutical agent is selected from ABT-089, ACC-001, Alzhemed, Ampalex, Ave-1625, AZD-103, Babineuzumab, CAD-106, Celebrex, D-cycloserine, Dimebon, Donepezil, D-serine, Flurizan, Galanthamine, Glycine, GSK-189254, GSK742457, Isopronicline, Lecozotan, Leuprolide, m266, MEM-3454, Memantine, Neramexane, NP-031122, Ono-2506, Pioglitazone, Posiphen, PRX-03140, Riluzole, Rivastigmine, Rosiglitazone, SGS-742.

6. The use of Claim 1, **characterized in that** the medicament comprises the peptide or a pharmaceutically acceptable addition salt thereof, alone or in combination with one or more pharmaceutically acceptable carrier and/or excipient.

7. The use of Claim 3, **characterized in that** the medicament comprises the peptide or a pharmaceutically acceptable addition salt thereof, alone or in combination with one or more pharmaceutically acceptable carrier and/or excipient.

8. Use of a peptide comprising amino acid sequence X-Y or Y-X, wherein X is an aromatic amino acid and Y is one or more additional amino acid other than glycine, the peptide having at least 2 amino acid residues and less than 15 amino acid residues, for the prevention and treatment of a condition **characterized by** central or peripheral neuronal insulin receptor impairment and insulin resistance associated with β-amyloid (Aβ) toxicity.

9. The use of Claim 8, **characterized in that** the condition **characterized by** central or peripheral neuronal insulin receptor impairment and insulin resistance is selected from the group consisting of amyotrophic lateral sclerosis, Alzheimer's disease, Parkinson's disease, Huntington's disease, schizophrenia, fronto-temporal dementia, Nieman-Pick disease Type C, obesity, depressive disorders, spinocerebellar ataxia, progressive supramuscular palsy, multiple sclerosis, Guillain-Barré syndrome, Charcot-Marie-Tooth syndrome, insulin receptor impairment, Cushing's disease, Donohue syndrome, polycystic ovarian syndrome, haemochromatosis, hypertension, HIV infection and tumors.

10. The use of the peptide of Claim 8, in combination with at least one additional pharmaceutical agent which is **characterized** as being effective in treating progressive neurodegenerative and metabolic disorders.

11. The use of Claim 10, wherein the at least one additional pharmaceutical agent is selected from medications **characterized** as being useful in preventing and treating progressive neurodegenerative and metabolic disorders, such medications being selected from other β-amyloid aggregation inhibitors (GAG inhibitors, β-sheet inhibitors, QC inhibitors), β-amyloid inhibitors (e.g. γ-secretase inhibitors, β-secretase inhibitors, α-secretase activators, glutaminyl-cyclase-inhibitors, APP expression inhibitors, passive and active vaccination / immunization), phosphorylated tau modulators (e.g. GSK-3 and CDK-5 inhibitors), PPARγ modulators, glutamate modulators (e.g. NMDA receptor antagonists, NR2B antagonists, glycine_{B} antagonists / agonists, AMPAkines, metabotropic glutamate receptor group I agonists / positive allosteric modulators, metabotropic glutamate receptor group II/III antagonists / negative allosteric modulators, glutamate release modulators), cannabinoid modulators (e.g. CB1 receptor antagonists, CB2 receptor agonists), GABA_{B} receptor antagonists, acetylcholine-esterase inhibitors, acetylcholine receptor modulators (α7 and α4β2 nicotinic receptor agonists, M1 receptors agonists, M2 receptor antagonists), serotonin modulators (e.g. 5-HT_{1A} and 5-HT₆ receptor antagonists, 5-HT₄ receptor agonists), GNRH agonists, histamine modulators (e.g. H1 and H3 receptor antagonists), apoptosis / necrosis inhibitors, Cox inhibitors, dopamine modulators (e.g. D1 receptor agonists), adenosine modulators (e.g. A1 / A2A receptor antagonists), phosphodiesterase modulators (e.g. PDE4 inhibitors), Cu²⁺/Zn²⁺ chelators, Ca²⁺ channel antagonists, statins, NSAIDs.

12. The use of Claim 10, wherein the at least one additional pharmaceutical agent is selected from ABT-089, ACC-001, Alzhemed, Ampalex, Ave-1625, AZD-103, Babineuzumab, CAD-106, Celebrex, D-cycloserine, Dimebon, Donepezil, D-serine, Flurizan, Galanthamine, Glycine, GSK-189254, GSK742457, Isopronicline, Lecozotan, Leuprolide, m266, MEM-3454, Memantine, Neramexane, NP-031122, Ono-2506, Pioglitazone, Posiphen, PRX-03140, Riluzole, Rivastigmine, Rosiglitazone, SGS-742.

13. A pharmaceutical composition comprising a peptide of Claim 8, or a pharmaceutically acceptable addition salt thereof, alone or in combination with one or more pharmaceutically acceptable carrier and/or excipient.
